# EUROPEAN PATENT APPLICATION

(11) **EP 2 524 644 A1**
(43) Date of publication of application: **21.11.2012**
(21) Application number: 10848007.0
(22) Date of filing: 28.12.2010
(51) Int. Cl.: A61B 1/00, A61B 1/12

(54) **ENDOSCOPIC TREATMENT DEVICE**

(30) Priority: 16.03.2010 US 724761
(71) Applicant: Terumo Cardiovascular Systems Corporation, Ann Arbor, Michigan 48103 (US); OLYMPUS MEDICAL SYSTEMS CORP., Tokyo 151-0072 (JP)
(72) Inventor: KANO, Akihito, Hachioji-shi Tokyo 192-8512 (JP); KADYKOWSKI, Randal James, Ann Arbor, Michigan 48103 (US); CHARRON-KELLER, Lyne Madeleine, Ann Arbor, Michigan 48103 (US)
(74) Representative: Schicker, Silvia
(86) International application number: PCT/JP2010/073703
(87) International publication number: WO 2011/114601

(57) **Abstract**

An endoscopic surgical instrument (41) includes an insertion section (42) to be inserted into a body cavity, a grip section (400) provided to be linked with a base end of the insertion section (42), a wiping section (417) that is provided at a tip end of the insertion section (42) and is pivoted to wipe off extraneous matter (418) adhering to an observation surface (54b) provided at a tip end part of a lens (54b), an axial member (500) that is inserted through the insertion section (42) and is connected to the wiping section (417), and an operation section (419) that is provided over a whole circumference of a tip end (400f) of the grip section (400) in a circumferential direction in a longitudinal direction of the grip section (400), is connected to the axial member (500), and operates the wiping section (417) through the axial member (500). A center axis (501a) of operation section (419) is coaxial to a center axis (501b) of an endoscope (51) when the endoscope (51) inserted through the grip section (400) and the insertion section (42).

## Description

### Technical Field

The present invention relates to an endoscopic surgical instrument for treating an object such as a blood vessel.

### Background Art

In recent cardiovascular bypass surgeries, there are cases of using as a bypass vessel, for example, a blood vessel in a lower limb which is a great saphenous vein of a patient or in an upper limb artery used as bypass such as radial artery of a patient. A living tissue harvesting surgical system is used to harvest a blood vessel under observation through an endoscope.

Such this living tissue harvesting surgical system includes an endoscopic surgical instrument for treating an object (living tissue) such as a blood vessel, and an endoscope which is inserted into the endoscopic surgical instrument. The endoscope has an observation surface (e.g., an objective lens) in an imaging system which picks up an image of the object. The endoscopic surgical instrument includes a wiper as a wiping section which wipes, for example, blood adhering to the objective lens.

Such surgical systems are disclosed in, for example, Patent Literature 1, Patent Literature 2, and Patent Literature 3.

Patent Literature 1 discloses an endoscopic vessel harvesting system which allows an operator to operate plural operation sections by fingers while gripping a sheath, and also allows the operator to carry out a single hand operation.

Patent Literature 2 discloses an endoscopic surgical instrument with excellent usability in which an endoscope is automatically wiped when to treat a tissue.

Patent Literature 3 discloses an endoscopic device comprising a wiper with excellent usability for a low price.

### Citation List

### Patent Literature

Patent Literature 1: Jpn. Pat. Appln. KOKAI Publication No. 2003-190171
Patent Literature 2: Jpn. Pat. Appln. KOKAI Publication No. 2007-37632
Patent Literature 3: Jpn. Pat. Appln. KOKAI Publication No. 2006-218240

### Disclosure of Invention

In general, an endoscopic surgical instrument has a wiper lever, which is an operation section used for operating a wiper. The operation section is arranged on the side opposite to that of the grip surface of a grip section with which the endoscopic surgical instrument is held. The operation section is hard to operate when the holder is being held because the operator's fingers may not reach the operation section. In addition, the operation section protrudes in a direction opposite to that of the grip surface. With this structure, the operation section may touch a portion (not shown), causing the wiper to operate in an undesirable manner, when the grip surface is being held.

The invention provides an endoscopic surgical instrument which has easy operation of wiper function when gripped by a hand.

According to an aspect of the invention, there is provided an endoscopic surgical instrument comprising an insertion section to be inserted into a body cavity, a grip section provided to be linked with a base end of the insertion section, a wiping section that is provided at a tip end of the insertion section and is pivoted to wipe off extraneous matter adhering to an observation surface provided at a tip end of an endoscope inserted through the grip section and the insertion section, an axial member that is inserted through the insertion section and is connected to the wiping section and an operation section that is provided over a whole circumference of a tip end of the grip section in a circumferential direction in a longitudinal direction of the grip section, is connected to the axial member, and operates the wiping section through the axial member, wherein a center axis of the operation section is coaxial to a center axis of the endoscope when the endoscope inserted through the grip section and the insertion section.

According to another aspect of the invention, there is provided an endoscopic surgical instrument comprising, an insertion section to be inserted into a body cavity, a grip section provided to be linked with a base end of the insertion section, a wiping section that is provided at a tip end of the insertion section and is pivoted to wipe off extraneous matter adhering to an observation surface provided at a tip end of an endoscope inserted through the grip section and the insertion section, an axial member that is inserted through the insertion section and is connected to the wiping section and an operation section that is provided at a tip end of the grip section, opens and closes in a radial direction of the grip section, is connected to the axial member, and operates the wiping section through the axial member by opening and closing, wherein a perpendicular axis which is perpendicular an open/close axis of the operation section and is provided along a longitudinal direction of the insertion section is coaxial to a center axis of the endoscope when the endoscope inserted through the grip section and the insertion section.

According to another aspect of the invention, there is provided an endoscopic surgical instrument comprising, an insertion section to be inserted into a body cavity, a grip section provided to be linked with a base end of the insertion section, a wiping section that is provided at a tip end of the insertion section and is pivoted to wipe off extraneous matter adhering to an observation surface, an axial member that is inserted through the insertion section and is connected to the wiping section and an operation section that is provided over a whole circumference of a tip end of the grip section in a circumferential direction in a longitudinal direction of the grip section, is connected to the axial member, and operates the wiping section through the axial member, wherein a center axis of the operation section and a center axis of the axial member are positioned on respectively different axes.

### Brief Description of Drawings

FIG. 1 illustrates a living tissue harvesting surgical system including an endoscopic surgical instrument according to the first embodiment of the invention;
FIG. 2 is a perspective view of a harvester;
FIG. 3 is a perspective view illustrating a configuration in a base end side of the harvester;
FIG. 4 is a perspective view illustrating a configuration of a tip end of the harvester (insertion section);
FIG. 5 is a perspective view illustrating the configuration of the tip end of the harvester (insertion section), for describing operation of a lock axis illustrated in FIG. 4;
FIG. 6 is a cross-sectional view illustrating an operational configuration of the harvester in a major axis direction of the harvester;
FIG. 7 is a view from an arrow 7 denoted in FIG. 4;
FIG. 8 is a cross-sectional view in the major axis direction, illustrating a gas supply configuration of the harvester;
FIG. 9 is a conceptual assembly view from an arrow 9 denoted in FIG. 6;
FIG. 10 is a view for describing forward/backward movement of a vein keeper lever and a vein keeper;
FIG. 11 is an endoscopic image of main blood vessel captured (secured) in the vein keeper;
FIG. 12 is also an endoscopic image a side branch and blood vessel interaction with vein keeper when said blood vessel is captured (secured) in the vein keeper;
FIG. 13A is a front view of a tip end of a grip section;
FIG. 13B illustrates a relationship in positions between a center axis of a wiper lever, a center axis of a rigid endoscope, a center axis of a rigid endoscope insertion channel, and a center axis of a wiper axis;
FIG. 13C illustrates movement of the wiper axis associated with pivot operation of the wiper lever;
FIG. 13D illustrates movement of the wiper axis associated with pivot operation of the wiper lever;
FIG. 13E is a perspective view illustrating a state in which the wiper lever is operated with the grip section gripped;
FIG. 13F illustrates a relationship between a pivot angle of the wiper lever and a pivot angle of the wiper axis;
FIG. 14A is a side view of a harvester in the second embodiment;
FIG. 14B illustrates a relationship in positions along a line 14B-14B denoted in FIG. 14A between a center axis of a pivot part, a center axis of a rigid endoscope, a center axis of a rigid endoscope insertion channel, and a center axis of a wiper axis;
FIG. 14C illustrates a relationship between a pivot angle of a pivot part and a pivot angle of a wiper axis;
FIG. 15A is a side view of a harvester in the third embodiment;
FIG. 15B illustrates a relationship between a forward/backward movement section and a pivot press section in a circle 15B denoted in FIG. 15A;
FIG. 15C is a front view of a grip section;
FIG. 15D is a top view of the forward/backward movement section and pivot press section;
FIG. 15E illustrates a relationship between the grip section, the forward/backward movement section, the pivot press section, and a wiper axis along a line 15E-15E denoted in FIG. 15A, e.g., a relationship in positions between a center axis of the pivot press section, a center axis of a rigid endoscope, a center axis of a rigid endoscope insertion channel, and a center axis of the wiper axis;
FIG. 15F illustrates a relationship between a pivot angle of a wiper lever and a pivot angle of the wiper axis;
FIG. 16A is a side view of a harvester in the fourth embodiment;
FIG. 16B is a front view of a grip section;
FIG. 16C is a cross-sectional view along a line 16C-16C illustrated in FIG. 16A;
FIG. 16D is a cross-sectional view along a line 16D-16D illustrated in FIG. 16C; and
FIG. 16E illustrates a relationship between frames, a pin, and a groove, viewed from an arrow 16E in FIG. 16D.

### Best Mode for Carrying Out the Invention

Hereinafter, embodiments of the invention will be described in details with reference to the drawings.

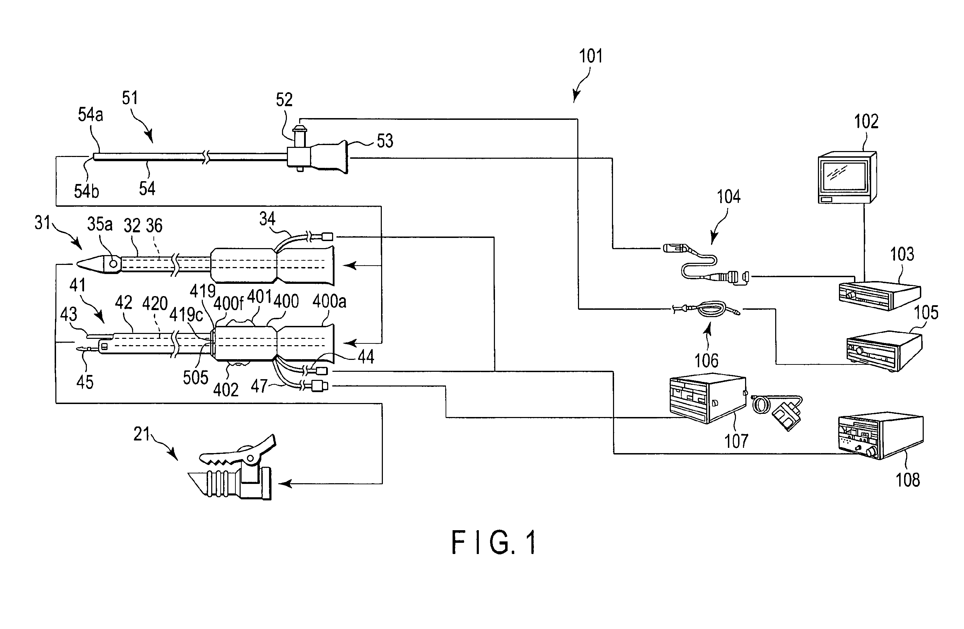

The first embodiment will now be described with reference to FIGS. 1, 2, 3, 4, 5, 6, 7, 8, 9, 10, 11, 12, 13A, 13B, 13C, 13D, 13E and 13F.

In the embodiments below, the term of object (a living tissue including a tissue to be harvested) is, for example, a blood vessel 11 in a body cavity, an incised side branch 11a of a blood vessel, or a bleeding point positioning on a wall part in a body cavity. By "surgery" is meant incision, excision, perforation, exfoliation, coagulation, stopping bleeding, harvesting, cauterization, cutting, etc.

By "circumferential direction" is meant a circumferential direction in a longitudinal direction of a grip section 400.

FIG. 1 illustrates a surgical system 101 for harvesting a living tissue (hereinafter simply referred to as a surgical system), which includes an endoscopic surgical instrument described later according to the first embodiment.

For example in cardiac bypass surgery, a blood vessel as an object is used as a bypass vessel. This blood vessel is used as, for example, a bypass and is a great saphenous vein as a blood vessel to be harvested (hereinafter simply referred to as a blood vessel), which extends from a femoral region in a lower limb to an ankle. This blood vessel is, for example, upper limb artery such as radial artery. This blood vessel is harvested over the whole length thereof by an endoscopic surgical instrument.

As illustrated in FIG. 1, the surgical system 101 includes a trocar 21, a dissector 31 as a living tissue exfoliation device, a living tissue cutting tool, i.e., a harvester 41 as an endoscopic surgical instrument, and a rigid endoscope 51 as an endoscope.

The surgical system 101 further includes a television monitor 102 as a display device, a camera control unit (hereinafter CCU) 103 connected to the television monitor 102, a television camera cable 104 connected to the CCU 103, a light source device 105 which emits light, a light guide cable 106 connected to the light source device 105, an electro surgical generator device 107, and a gas supply device 108 which supplies a desired gas, such as a carbon dioxide gas.

The dissector 31 and harvester 41 are configured to allow the rigid endoscope 51 to be inserted in. An operator harvests a blood vessel while viewing an endoscopic image imaged by the rigid endoscope 51 on the television monitor 102.

The rigid endoscope 51 will now be described.

A light guide connector part 52 and an eyepiece part 53 are provided on a base end side of the rigid endoscope 51.

An end of the light guide cable 106 is connected to the light guide connector part 52. The other end of the light guide cable 106 is connected to the light source device 105. A light guide such as a light fiber is inserted in the light guide cable 106. The light emitted from the light source device 105 is supplied to the rigid endoscope 51 through the light guide cable 106. With this light, the rigid endoscope 51 illuminates inside of the object from a tip end part 54a of an insertion section 54, which is also a tip end part of the rigid endoscope 51.

The television camera cable 104 is connected to the eyepiece part 53. When the television camera cable 104 is connected to the CCU 103 and the CCU 103 is connected to the television monitor 102, an image of an object imaged by the rigid endoscope 51 is displayed on the television monitor 102.

On the tip end side of the rigid endoscope 51, the insertion section 54 is provided. This insertion section 54 is inserted from a base end side of the dissector 31 into a rigid endoscope insertion channel 36 described later of the dissector 31. The insertion section 54 is inserted into a rigid endoscope insertion channel 420 extending through an insertion section 42 (to be described later) of the harvester 41 from the base end side of the harvester 41.

The rigid endoscope 51 has an observation surface 54b (objective lens) in an unillustrated imaging system which picks up an image of the object in the tip end part 54a of the insertion section 54. An image of the object imaged through the observation surface 54b is displayed on the television monitor 102 by the television camera cable 104 and CCU 103.

The dissector 31 will be described next.

The dissector 31 is provided with an insertion section 32 to be inserted into a body cavity, a gas supply tube 34, and a rigid endoscope insertion channel 36 into which the insertion section 54 is inserted.

The gas supply tube 34 is connected to a gas supply tubing (not shown) which is connected to the gas supply device 108 and is supplied with a desired gas. This gas is discharged from an opening 35a provided at a tip end part of the insertion section 32 of the dissector 31. The rigid endoscope insertion channel 36 is inserted inside the dissector 31 along an axial direction of the dissector 31 from the base end side of the dissector 31 to the tip end part of the insertion section 32.

Next, the harvester 41 as the endoscopic surgical instrument according to the invention will be described with reference to FIGS. 1, 2, 3, 4, 5, 6, 7, 8, 9, 10, 11, 12, 13A, 13B, 13C, 13D, 13E and 13E.

The object is treated with the harvester 41 in a state in which the rigid endoscope 51 having the observation surface 54b as a window part at the tip end part 54a is inserted.

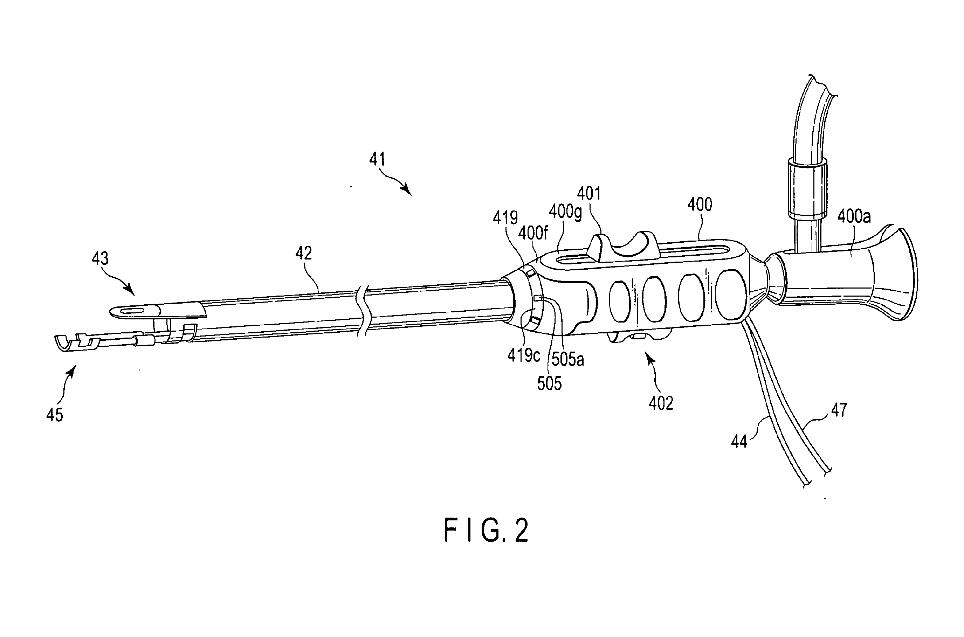

As illustrated in FIGS. 1 and 2, the harvester 41 includes the metal insertion section 42 to be inserted into a body cavity, and the grip section 400 to be linked to the base end of the insertion section 42 and allows to grip the harvester 41.

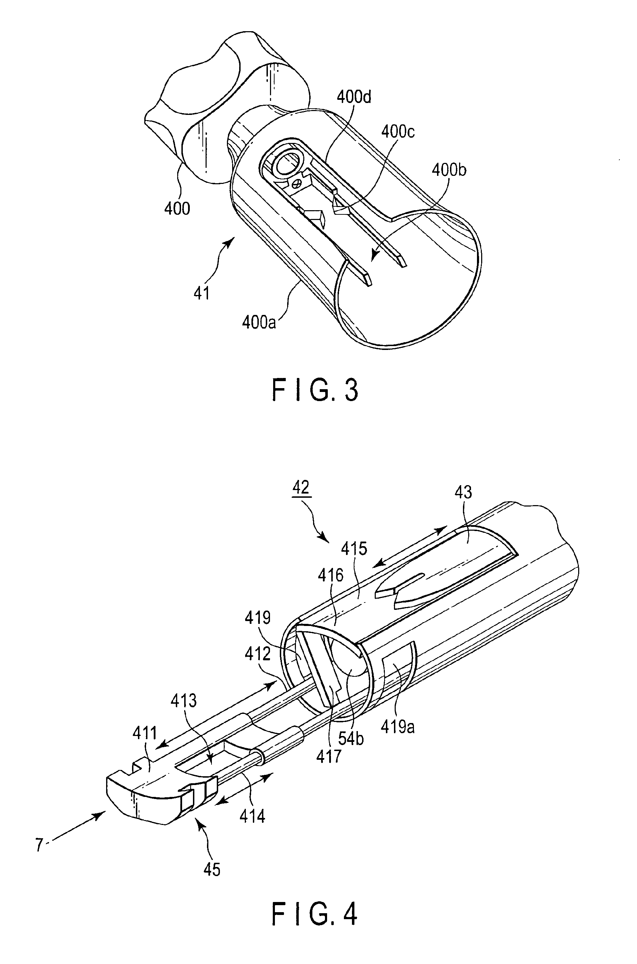

Further as illustrated in FIGS. 2 and 3, a base end part 400a of the grip section 400 as an endoscope holding section easily and steadily fixes the rigid endoscope 51 to the base end part (base end part 400a) of the harvester 41. On an inner circumferential surface of the base end part 400a, a guiding groove 400b is provided along an axial direction of the harvester 41. As illustrated in FIG. 3, a fixing member 400c is fixed to the guiding groove 400b by a screw. The fixing member 400c is formed by bending a metal plate member into a rectangular U-shape. Two ends of the U-shape each are bent so as to form a convex protruding inward into the U-shape.

Further, a notched part 400d is notched in the base end part 400a so as to allow the light guide connector part 52 to move along the notched part 400d when the rigid endoscope 51 is fixed to the harvester 41.

On the tip end side of the eyepiece part 53, an unillustrated convex is provided.

When the rigid endoscope 51 is inserted from the base end part 400a of the harvester 41, the unillustrated convex of the eyepiece part 53 moves along the guiding groove 400b and the light guide connector part 52 moves along the notched part 400d. At this time, if the rigid endoscope 51 is further inserted toward the grip section 400, the unillustrated convex of the eyepiece part 53 moves along inside the guiding groove 400b and passes through the convexes, resisting elastic force of the fixing member 400c.

That is, a positional relationship between the harvester 41 and the rigid endoscope 51 is set in a manner that, when the rigid endoscope 51 is inserted from the base end part side of the harvester 41, the light guide connector part 52 enters into the notched part 400d and the unillustrated convex of the eyepiece part 53 enters into the guiding groove 400b. As the rigid endoscope 51 is further inserted into the harvester 41, the unillustrated convex of the eyepiece part 53 is engaged in the fixing member 400c, sandwiched between the two ends thereof, and is prevented from easily falling out, owing to the elastic force of the fixing member 400c.

In this manner, the rigid endoscope 51 is fixed to the harvester 41.

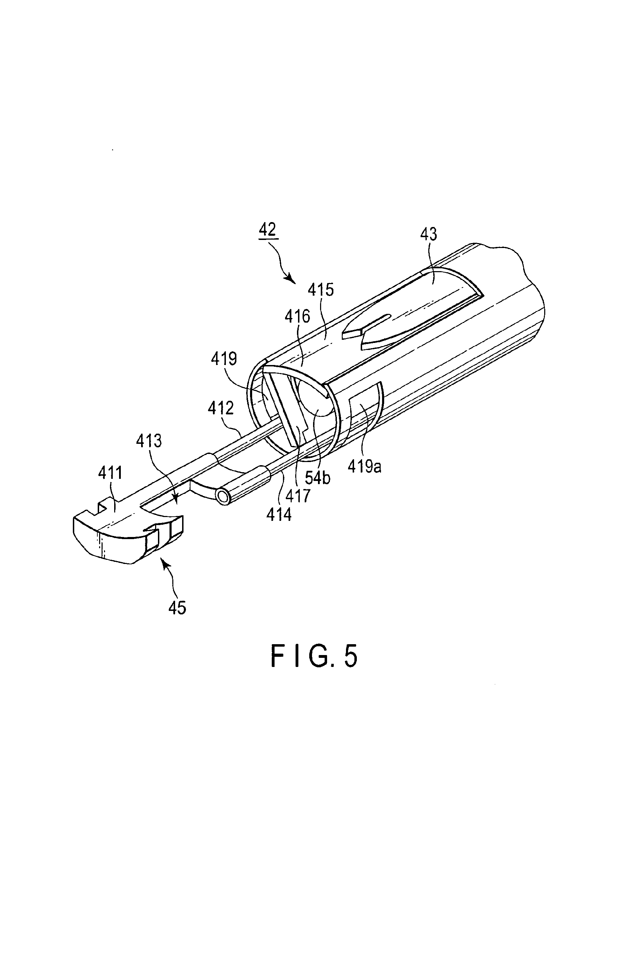

The insertion section 42 will be described next with reference to FIGS. 4 and 5.

A bipolar cutter 43 is provided on the upper portion of the tip end of the insertion section 42. A vein keeper 45, which is a holding member, is provided inside the lower portion of the tip end of the insertion section 42.

As illustrated in FIGS. 4 and 5, the vein keeper 45 includes a vein keeper axis 412 and a lock axis 414. The vein keeper axis 412 holds an almost U-shaped blood vessel holding base 411 to be movable forward and backward in the longitudinal direction of the insertion section 42. The lock axis 414 is provided parallel to the vein keeper axis 412 and moves forward and backward in the longitudinal direction of the insertion section 42 relative to the blood vessel holding base 411 to form, in the almost U-shaped blood vessel holding base 411, a closed space 413 which stores a blood vessel. As shown in FIGS. 4, 5 and 6, the vein keeper axis 412 and the lock axis 414 extend through the insertion section 42 and the grip section 400.

As illustrated in FIG. 4, the lock axis 414 forms the closed space 413 while being locked to the blood vessel holding base 411 like the vein keeper axis 412. Releasing the locked state of the lock axis 414 will open the closed space 413, as shown in FIG. 5. The lock axis 414 then moves forward and backward in the longitudinal direction of the insertion section 42 so as to allow a blood vessel 11 to be stored in the closed space 413.

The bipolar cutter 43 includes an unillustrated cutter body made of, for example, synthetic resin forming a transparent insulating member such as polycarbonate, an unillustrated voltage applied electrode as a first electrode which forms one of two bipolar electrodes, an unillustrated feedback electrode as a second electrode which forms the other of the two bipolar electrodes, unillustrated two leads and an unillustrated lead cover. The bipolar cutter 43 has a layered structure consisting of three layers, i.e., the feedback electrode as an upper layer, a side branch holding member, and the voltage applied electrode.

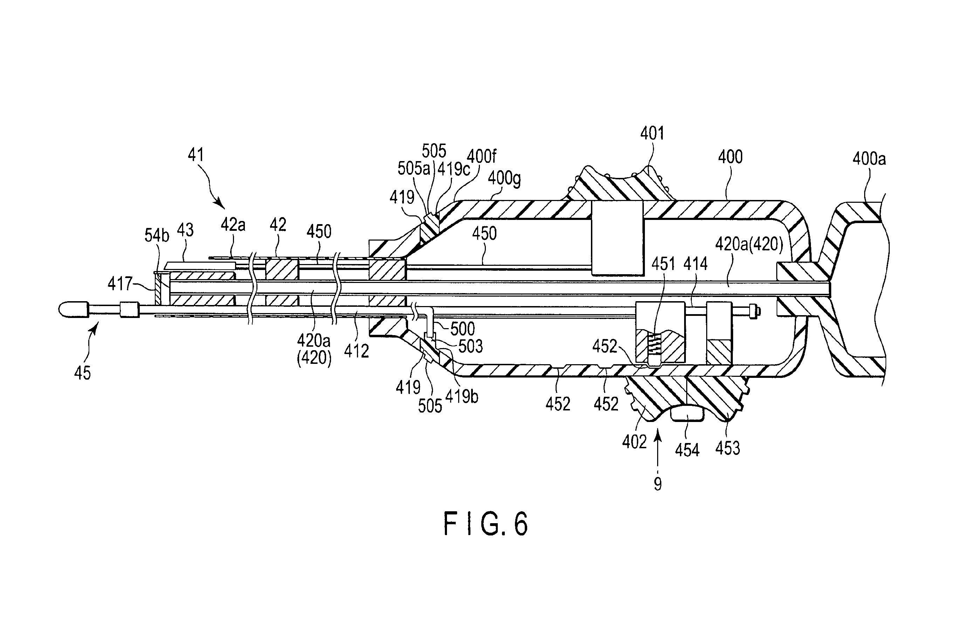

As illustrated in FIGS. 4 and 5, a notch 415 for receiving the bipolar cutter 43 is formed in the upper surface of the top side of the insertion section 42. As illustrated in FIG. 6, a bipolar axis 450 for the forward/backward movement of the bipolar cutter 43 is connected to the bipolar cutter 43. The bipolar axis 450 is inserted into the insertion section 42 through the notched part 415. A guard part 416 having an arc-shaped cross section is provided on the inner wall surface of the notched part 415.

As illustrated in FIGS. 4, 5, 6 and 7, a wiper 417 is provided on the inner surface (inside) of a tip end of the insertion section 42. The wiper 417 is a wiping section which pivots to wipe off extraneous matter 418 adhering to the observation surface 54b provided at the tip end part 54a of the rigid endoscope 51. As shown in FIG. 6, a wiper axis 500 as a rod-like axial member, which is connected to the wiper 417 and is inserted through the insertion section 42.

While one end of the wiper 417 serves as an axis, the other end of the wiper 417 wipes on the inside of the guard part 416, thereby forming a wiper guard part.

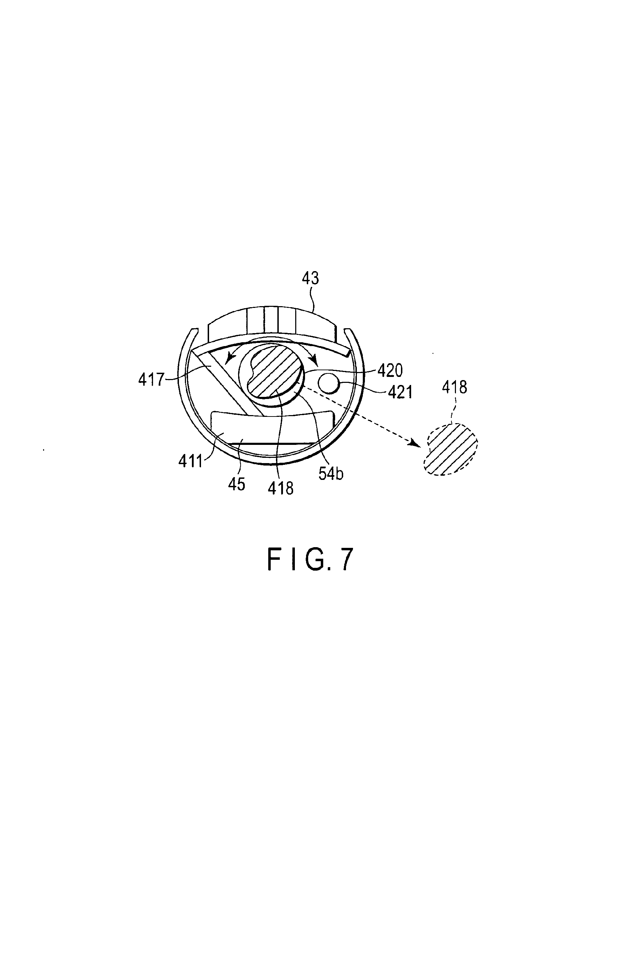

Further, a sweeping hole 419a for sweeping the extraneous matter 418 (see FIG. 7) wiped by the wiper 417 is provided at a part of the cylindrical wiper guard part, as illustrated in FIGS. 4 and 5. The extraneous matter 418 may be, for example, blood, fat, smoke generated by the electro surgical generator device, etc.

As illustrated in FIG. 7, an opening of the rigid endoscope insertion channel 420 in which the rigid endoscope 51 is inserted and an opening of a gas supply channel 421 for supplying a gas are provided adjacent to each other, at a desired position inside a tip end surface of the insertion section 42.

Next, the grip section 400 will be described with reference to FIGS. 1, 2, 6, and 8.

As illustrated in FIGS. 1 and 2, the grip section 400 is provided with an electrical cable 47 for the bipolar cutter 43, and a gas supply tube 44.

The electrical cable 47 is connected to the electro surgical generator device 107 by a connector provided at a base end of the cable 47.

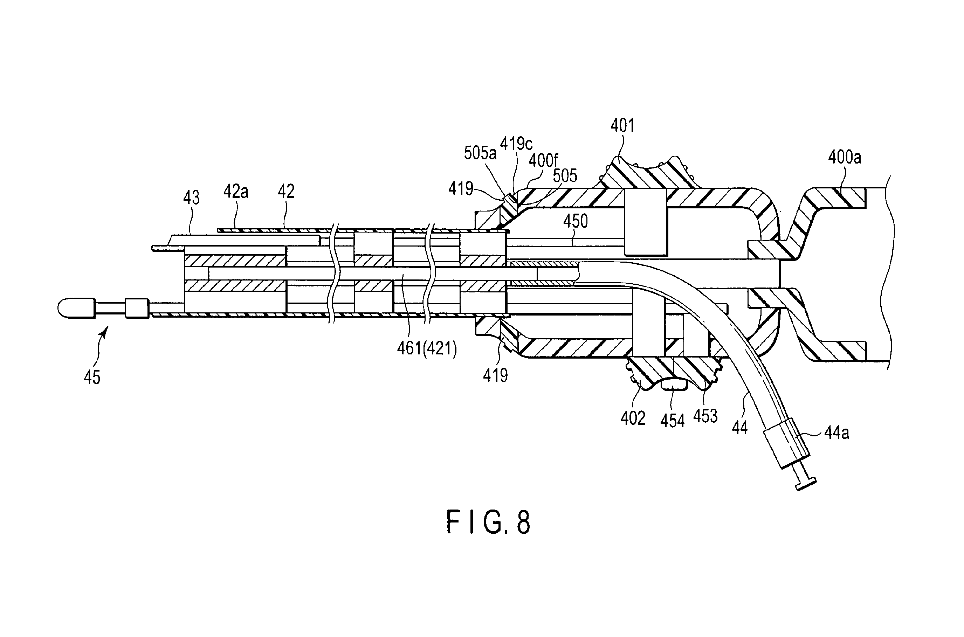

A gas supply connector 44a is provided at a base end of the gas supply tube 44. The gas supply connector 44a is connected to the gas supply tubing (not shown) which is connected to the gas supply device 108. At this time, the gas supply tube 44 is supplied with a desired gas from the gas supply device 108 via the gas supply tubing. The desired gas is, for example, a carbon dioxide gas as described previously. In the grip section 400, an end of a gas supply tubing 461 is engaged in the gas supply tube 44. As illustrated in FIG. 8, the gas supply tubing 461 is inserted along an axial direction of the harvester 41 from the base end side of the grip section 400 to the tip end part 42a of the insertion section 42, inside the harvester 41. The gas supply tubing 461 is made of metal which forms a gas supply channel 421. The desired gas supplied from the gas supply device 108 is discharged from an opening of the gas supply channel 421 through the gas supply tube 44 and gas supply tubing 461.

As illustrated in FIGS. 1 and 6, a metal tube member 420a forming the rigid endoscope insertion channel 420 is inserted along the axial direction of the harvester 41 from the base end side of the grip section 400 to the tip end part of the insertion section 42, inside the harvester 41.

As illustrated in FIGS. 1, 2, and 6, the grip section 400 is provided with a bipolar cutter lever 401 which can move forward and backward in the longitudinal direction of the grip section 400 in order to operate the bipolar cutter 43.

As illustrated in FIG. 6, the bipolar axis 450 which is inserted through the insertion section 42 and grip section 400 and is connected to the bipolar cutter 43 is connected to the bipolar cutter lever 401. That is, the bipolar cutter 43 is connected to the bipolar cutter lever 401 by the bipolar axis 450 inserted through the insertion section 42.

When the bipolar cutter lever 401 moves forward and backward in the longitudinal direction of the grip section 400, the bipolar cutter 43 moves forward and backward through the insertion section 42 through the bipolar axis 450 in association with movement of the bipolar cutter lever 401. In other words, when the bipolar cutter lever 401 moves forward and backward along the longitudinal direction of the grip section 400, force of the forward and backward movement is transmitted through the bipolar axis 450 to the bipolar cutter 43, which accordingly moves forward and backward.

As illustrated in FIGS. 1, 2, and 6, the grip section 400 is provided with a vein keeper lever 402 which is movable forward and backward in the longitudinal direction of the grip section 400, in order to operate the vein keeper 45.

As shown in FIG. 6, the vein keeper axis 412 described above which is inserted through the insertion section 42 and the grip section 400 and is connected to the vein keeper 45 is connected to the vein keeper lever 402. That is, the vein keeper 45 is connected to the vein keeper lever 402 through the vein keeper axis 412 inserted through the insertion section 42.

When the vein keeper lever 402 moves forward and backward in the longitudinal direction of the grip section 400, the vein keeper 45 moves forward and backward through the vein keeper axis 412 in association with the forward and backward movement of vein keeper lever 402. In other words, as the vein keeper lever 402 moves forward and backward along the longitudinal direction of the grip section 400, force of the forward and backward movement is transmitted through the vein keeper axis 412 to the vein keeper 45, which accordingly moves relative to the front of the insertion section 42.

On an inner surface (inside) of the grip section 400, there is provided a click assembly 451 which holds the vein keeper lever 402 and vein keeper axis 412 and fixes positions of the vein keeper lever 402 and vein keeper axis 412.

In association with integral movement of the vein keeper lever 402 and vein keeper axis 412, the click assembly 451 moves on the inner surface of the grip section 400. At this time, the click assembly 451 is positioned at any of, for example, three click grooves 452 provided in the inner surface of the grip section 400, and pin-presses the inner surface (at the click groove 452) of the grip section 400. At the position of the click groove 452, the vein keeper lever 402 and vein keeper axis 412 are stably fixed by the click assembly 451 which pin-presses the click groove 452.

However, if any force acts on the vein keeper lever 402 in the longitudinal direction, the click assembly 451 then easily comes out of the click groove 452.

As illustrated in FIG. 6, the grip section 400 is provided with a lock lever 453 which is detachably and attachably connected to the vein keeper lever 402, and a lock button 454 which is pressed down to separate the vein keeper lever 402 and the lock lever 453 away from each other.

The lock lever 453 is connected to the lock axis 414. When the lock lever 453 moves forward and backward, maintained separate from the vein keeper lever 402, the lock axis 414 moves forward and backward, thereby allowing the closed space 413 to contain the blood vessel 11, as illustrated in FIGS. 4 and 5.

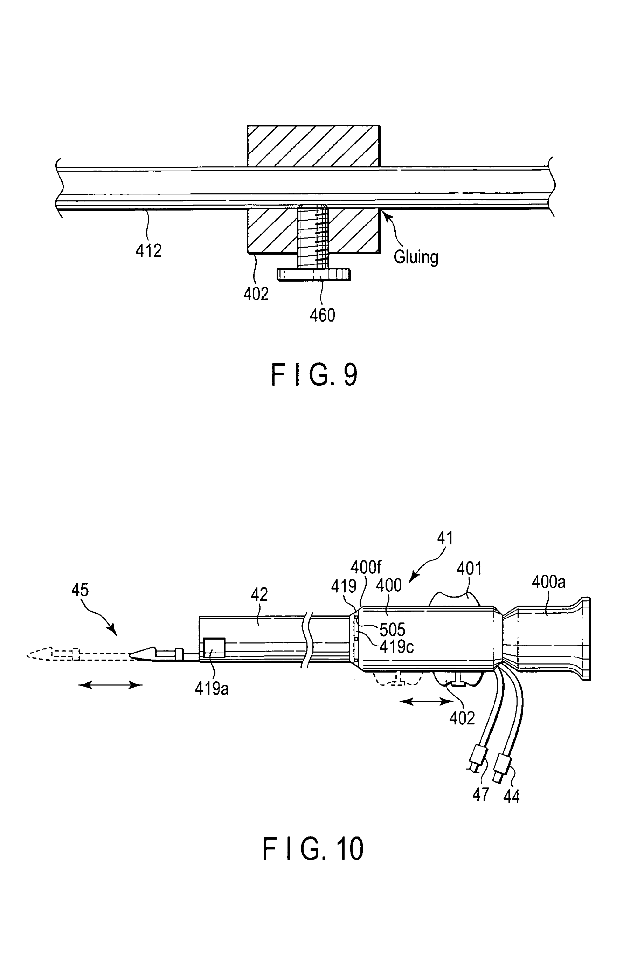

As illustrated in FIG. 9, the vein keeper lever 402 is firmly fixed to the vein keeper axis 412 by the screw 460 and by bonding.

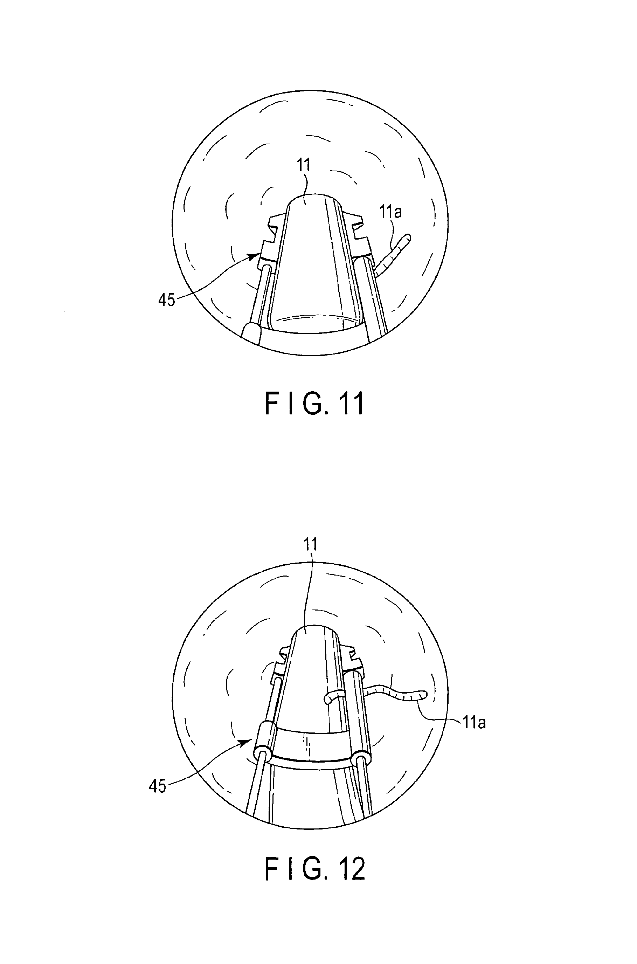

In this embodiment as described above, when the vein keeper lever 402 moves forward and backward, the vein keeper 45 moves forward and backward relative to the front of the insertion section 42, as illustrated in FIG. 10. Therefore, for example, if conditions of the side branch 11a are difficult to check on an endoscopic image as illustrated in FIG. 11 when cutting the side branch 11a, the vein keeper lever 402 is moved forward in the longitudinal direction. Accordingly, the vein keeper lever 402 also moves forward from the tip end as illustrated in FIG. 12, and an endoscopic image as illustrated in FIG. 12 can be viewed suitably for visually checking conditions of the side branch 11a.

As illustrated in FIGS. 2, 6, and 13A, the grip section 400 is provided with a wiper lever 419 as an operation section which is provided over the whole circumference of a tip end 400f of the grip section 400 in the circumferential direction in the longitudinal direction of the grip section 400, and is connected to the wiper axis 500 thereby to operate the wiper 417 through the wiper axis 500.

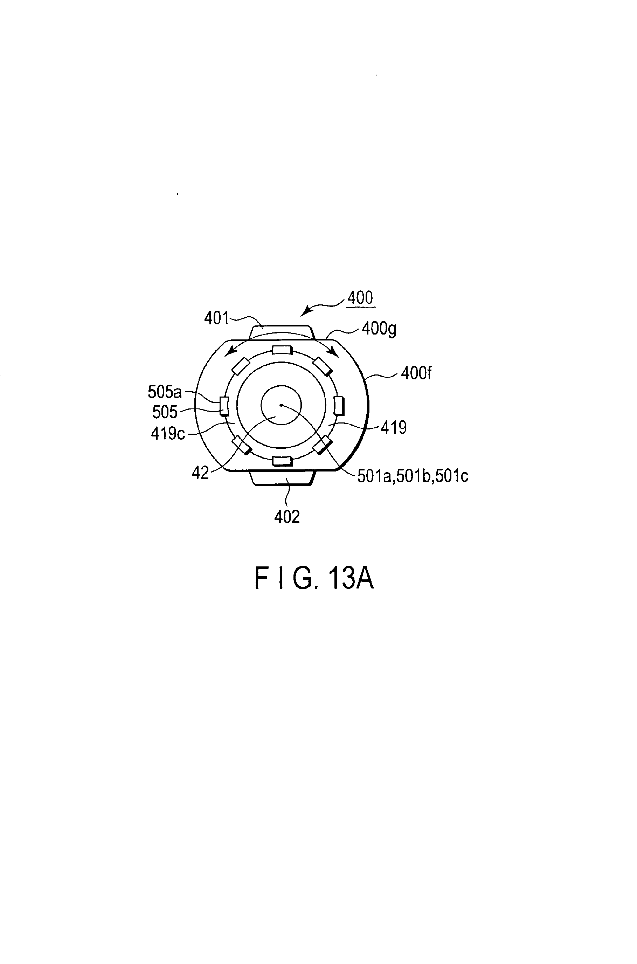

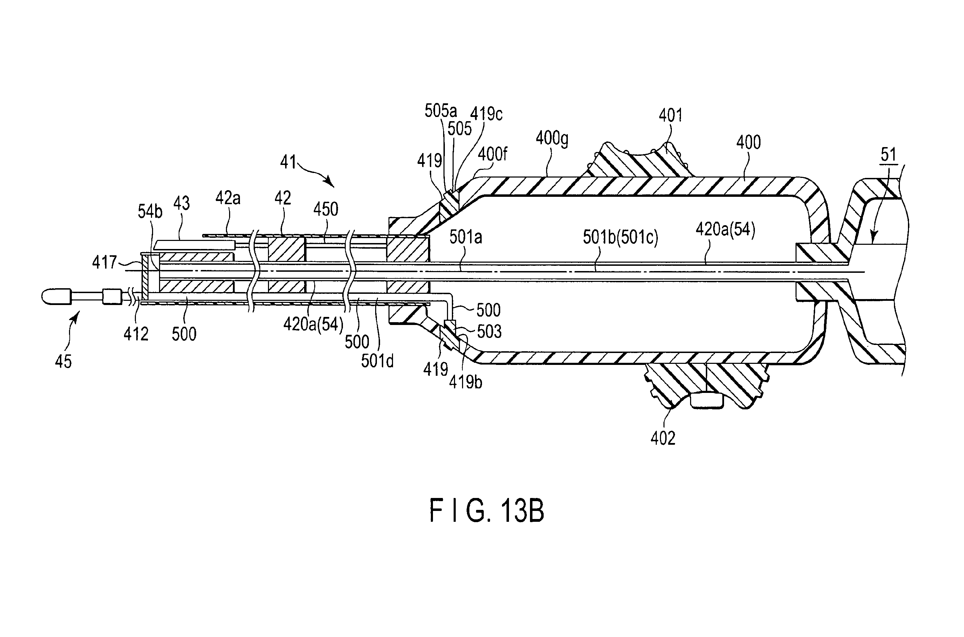

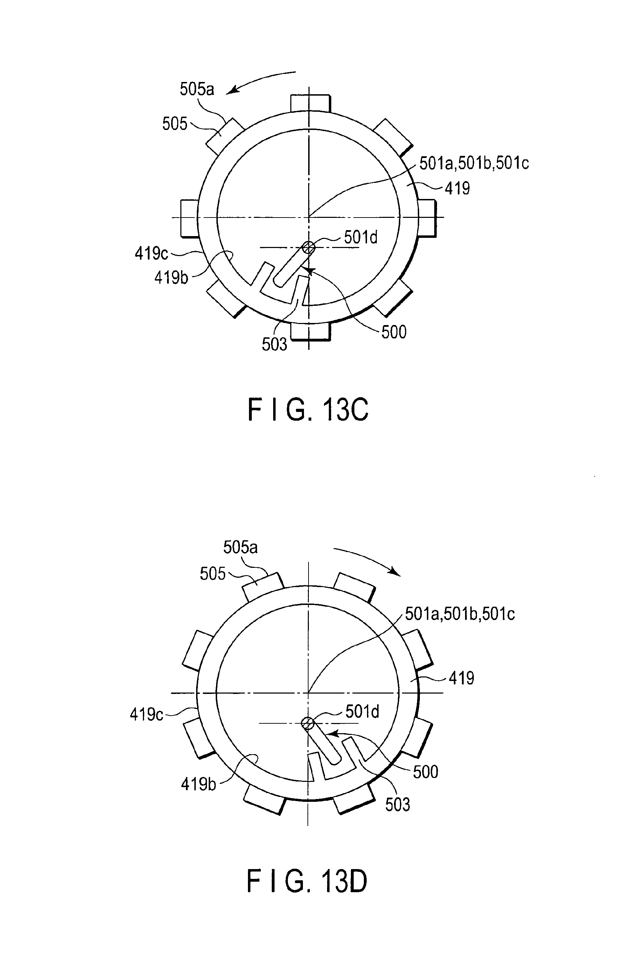

As illustrated in FIGS. 6, 13B, 13C, and 13D, a center axis 501a of the wiper lever 419 is coaxial to a center axis 501b of the rigid endoscope 51 inserted in the harvester 41 when the rigid endoscope 51 inserted through the harvester 41. In case of this embodiment, the center axis 501a of the wiper lever 419 is coaxial to a center axis 501c of the rigid endoscope insertion channel 420 in which the rigid endoscope 51 is inserted through.

Although details will be described later, as illustrated in FIGS. 13B, 13C, and 13D, the center axis 501a of the wiper lever 419 and a center axis 501d of the wiper axis 500 are positioned on respectively different axes.

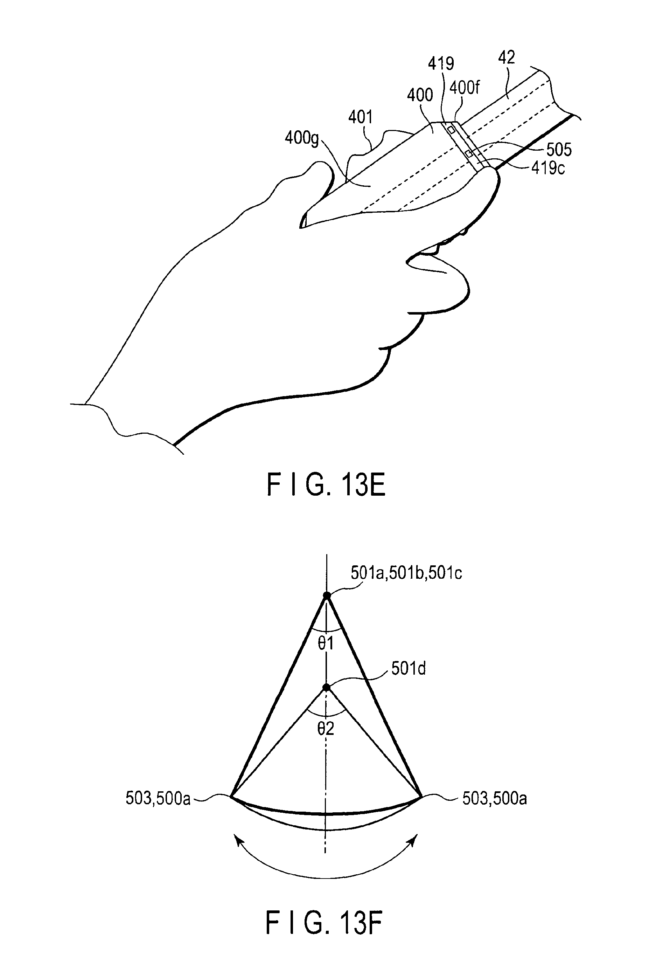

The wiper lever 419 is provided at a position where the wiper lever 419 can be operated with the grip section 400 gripped, as illustrated in FIG. 13E. This position designates the tip end 400f.

The wiper lever 419 in this embodiment can be pivoted in the circumferential direction, as illustrated in FIG. 13A. The wiper 417 is operated by pivoting the wiper lever 419 in the circumferential direction and thereby causing the wiper axis 500 connected to the wiper lever 419 to be also pivoted in the circumferential direction. At this time, a pivot angle θ1 of the wiper lever 419 is smaller than a pivot angle θ2 of the wiper axis 500, as illustrated in FIG. 13F (details thereof will be described later).

The wiper lever 419 includes a holder section 503 for holding the wiper axis 500 on an inner circumferential surface 419b, in order to connect to the wiper axis 500 as illustrated in FIGS. 13C and 13D. This holder section 503 is a cam which holds the wiper axis 500 by pinching an end of the wiper axis 500.

That is, the wiper lever 419 is engaged with the wiper axis 500 on the inner circumferential surface 419b. The other end of the wiper axis 500 is connected to the wiper 417. Thus, the wiper lever 419 is connected to the wiper 417 through the wiper axis 500 which is inserted through the insertion section 42.

As described above, when the wiper lever 419 is pivoted in the circumferential direction, pivot force thereof is transmitted to the wiper 417 through the holder section 503 and wiper axis 500, thereby to pivot the wiper 417 for wiping.

When the wiper lever 419 is thus pivoted in the circumferential direction, the wiper 417 is pivoted through the wiper axis 500 in association with the pivot operation.

On a cross-section of the wiper lever 419, the center axis 501a of the wiper lever 419 (axial center for pivoting) and the center axis 501d of the wiper axis 500 (axial center for pivoting) are located on respectively different axes, as illustrated in FIGS. 13C and 13D, in order that, even if the wiper lever 419 is pivoted by a desired pivot amount, the wiper axis 500 is pivoted by a pivot amount not smaller than the desired pivot amount, thereby to pivot the wiper 417 by a pivot amount not smaller than the desired pivot amount.

More specifically, as illustrated in FIG. 13F and as described above, the pivot angle θ1 of the wiper lever 419 (wiper 417) is smaller than the pivot angle θ2 of the wiper axis 500. That is, the pivot angle θ1 between the center axis 501a of the wiper lever 419 and the holder section 503 as a tip end part of the wiper lever 419 pivoted is smaller than the pivot angle θ2 between the center axis 501d of the wiper axis 500 and a tip end part 500a of the wiper axis 500 pivoted in a radial direction.

As illustrated in FIGS. 1, 2, and 6, the wiper lever 419 has a tapered shape. The wiper lever 419 does not protrude to a side opposite to a grip surface. Since the wiper lever 419 is provided at the tip end 400f of the grip section 400, the wiper lever 419 is provided closer to the tip end than the grip surface which is gripped by the operator. As illustrated in FIG. 13E, the grip surface designates an outer surface 400g of the grip section 400 in the base end part 400a of the grip section 400, the bipolar cutter lever 401, and the vein keeper lever 402.

The wiper lever 419 includes plural protrusions 505 as nubs for operating the wiper lever 419, which are provided on an outer circumferential surface 419c of the wiper lever 419 over the circumferential direction thereof. In this embodiment, the wiper lever 419 including the protrusions 505 is exposed outside. The protrusions 505 are provided, for example, at equal intervals along the circumferential direction and each have a convex shape.

In a radial direction of the grip section 400, tip ends 505a of the protrusions 505 do not protrude out of the outer surface 400g of the grip section 400 but are located inside the outer surface 400g, as illustrated in FIG. 13A.

Described next will be an operation method according to this embodiment.

As illustrated in FIG. 13B, the insertion section 54 is inserted through the rigid endoscope insertion channel 420, and the rigid endoscope 51 is provided in the harvester 41.

As illustrated in FIG. 13E, the grip section 400 is gripped. In this state, the wiper lever 419 provided over the whole circumference of the tip end 400f of the grip section 400 is operated by the protrusions 505.

At this time, the protrusions 505 held by the operator are pivoted along the circumferential direction, as illustrated in FIG. 13A. Then, the wiper lever 419 is pivoted along the circumferential direction, as illustrated in FIGS. 13C and 13D. In accordance with the pivot operation, the wiper 417 is pivoted through the holder section 503 and wiper axis 500, as illustrated in FIG. 7. At this time, the wiper 417 wipes extraneous matter 418 adhering to the observation surface 54b, as illustrated in FIG. 7.

As illustrated in FIGS. 13C and 13D, the center axis 501a of the wiper lever 419 and the center axis 501d of the wiper axis 500 are positioned on respectively different axes. As illustrated in FIG. 13F, the pivot angle θ1 of the wiper lever 419 (wiper 417) is smaller than the pivot angle θ2 of the wiper axis 500. Therefore, when the wiper lever 419 is pivoted by a desired pivot amount, the wiper axis 500 is pivoted by a pivot amount not smaller than the desired pivot amount, thereby the wiper 417 connected to the wiper axis 500 is pivoted by a pivot amount not smaller than the desired pivot amount. That is, if the wiper lever 419 is provided by a desired angle, the wiper axis 500 and wiper 417 are pivoted by an angle greater than the desired angle.

Thus, the wiper 417 is pivoted over a wide range even with a small operation amount of the wiper lever 419. In this manner, the wiper 417 wipes the extraneous matter 418 adhering to the wide range of the observation surface 54b.

Thus, in this embodiment, the wiper lever 419 is provided over the whole circumference of the tip end 400f of the grip section 400, so as to be pivotable in the circumferential direction. Therefore, even when the harvester 41 as an endoscopic surgical instrument is gripped, the wiper lever 419 for operating the wiper 417 can be easily operated.

In this manner, in this embodiment, labor on the operator can be reduced, and operation time can be shortened.

In this embodiment, the center axis 501a of the wiper lever 419 and the center axis 501d of the wiper axis 500 are positioned on respectively different axes. The pivot angle θ1 of the wiper lever 419 (wiper 417) is set smaller than the pivot angle θ2 of the wiper axis 500. Therefore, in this embodiment, the wiper 417 can be pivoted over a wide range with a small operation amount of the wiper lever 419, and extraneous matter 418 adhering to the observation surface 54b can be wiped off over the wide range by the wiper 417.

Also in this embodiment, even if the pivot amount (operation amount) of the wiper lever 419 is small, the extraneous matter 418 adhering to the observation surface 54b can be wiped off over the wide range. Therefore, labor of operation for pivoting the wiper lever 419 can be saved.

Also in this embodiment, the wiper lever 419 can be operated with the grip section 400 gripped, as illustrated in FIG. 13E. Therefore, fatigue of the operator can be reduced, and operation time can be shortened.

Also in this embodiment, the wiper lever 419 and wiper axis 500 can be connected by the holder section 503. Therefore, the pivot amount of the wiper lever 419 can be transmitted to the wiper axis 500 without waste.

Also in this embodiment, the wiper lever 419 is configured to have a tapered shape. Therefore, operability of the wiper lever 419 can be improved when the grip section 400 is gripped.

Also in this embodiment, the wiper lever 419 is provided with the protrusions 505 as nubs arranged on the outer circumferential surface 419c in the circumferential direction. The wiper lever 419 including the protrusions 505 is therefore exposed outside. Therefore, operability of the wiper lever 419 can be improved more.

Also in this embodiment, the tip ends 505a are not protruded beyond the outer surface 400g in the radial direction of the grip section 400 but are located inside the outer surface 400g. Operability of the wiper lever 419 can be improved more. In this manner, the protrusions 505 are prevented from making contact with any unillustrated portion and from thereby causing an operation error of the wiper 417.

Also in this embodiment, the wiper lever 419 is provided at the tip end 400f of the grip section 400 having a tapered shape and does not protrude to a side opposite to a grip surface, so as to be pivotable along the circumferential direction. Therefore, in this embodiment, when the grip section 400 is gripped, the wiper lever 419 is prevented from making contact with unillustrated portions and from thereby causing an operation error of the wiper 417.

Also in this embodiment, the harvester 41 as an endoscopic surgical instrument and the rigid endoscope 51 as an endoscope are separated with each other, but the harvester 41 and the rigid endoscope 51 may be integrated with each other.

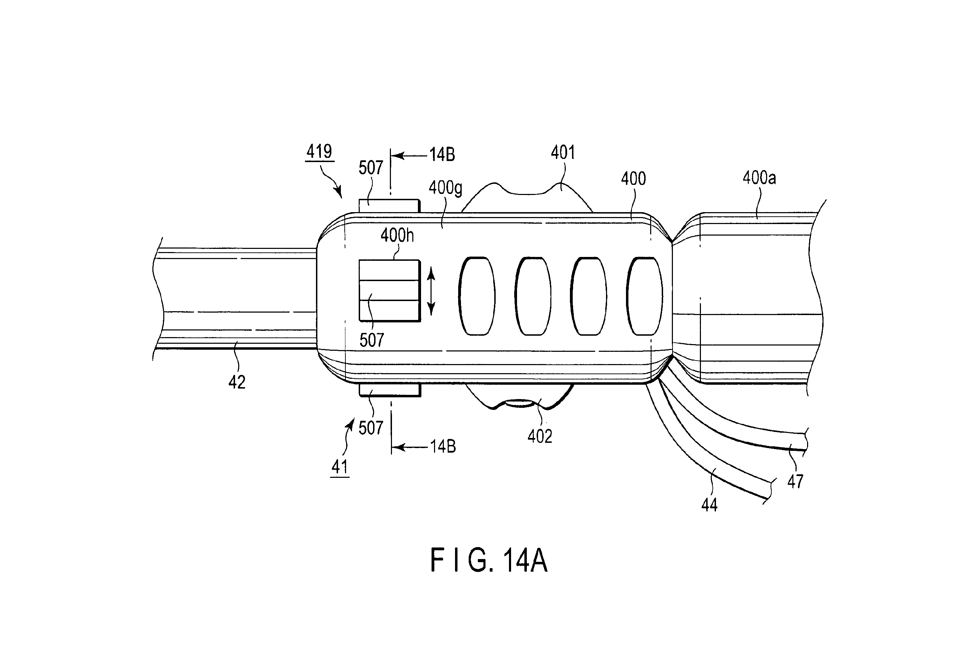

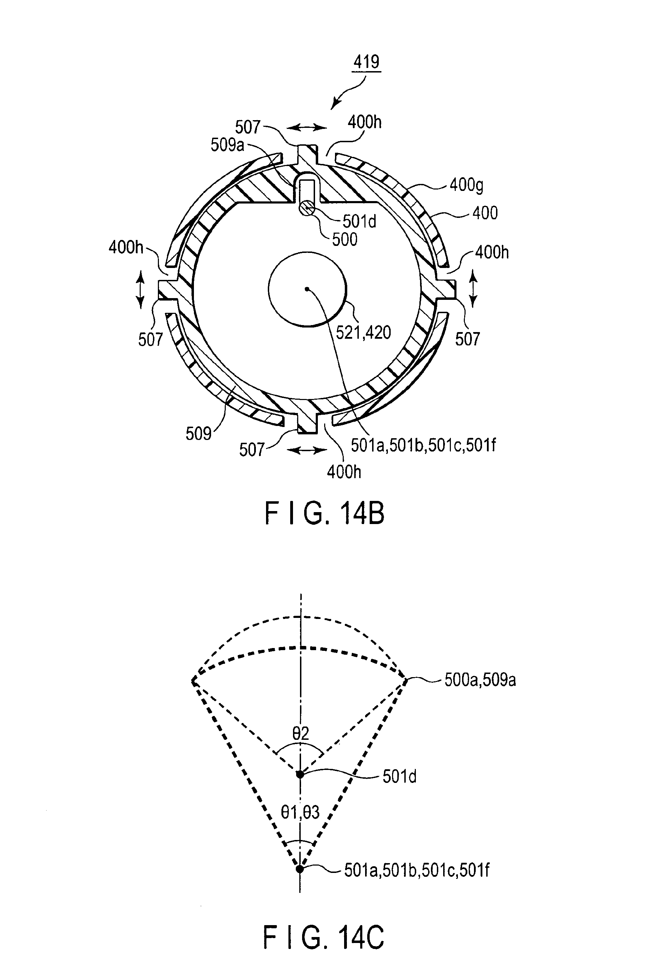

Next, the second embodiment of the invention will be described with reference to FIGS. 14A, 14B, and 14C. The same parts of the configuration as those of the first embodiment described above will be denoted at the same reference symbols, and omitted from descriptions given below.

As illustrated in FIGS. 14A and 14B, a wiper lever 419 according to this embodiment includes plural protrusions 507 which are exposed from an outer surface 400g, provided over the circumferential direction, form knobs for operating the wiper lever 419, and are pivotable in the circumferential direction and a pivot part 509 which includes a concave 509a for holding the wiper axis 500 and is integrated with the protrusions 507, provided inside the grip section 400, and is pivoted in association with pivot operation of the protrusions 507, thereby to pivot the wiper axis 500 through a concave 509a and to accordingly operate a wiper 417.

The protrusions 507 are provided, for example, at four positions at equal intervals in the circumferential direction.

Also, a grip section 400 includes openings 400h from which the protrusions 507 protrude. The openings 400h constrain pivoting of the protrusions 507 in the circumferential direction.

In this embodiment, a center axis 501f of the pivot part 509 which serves also a center axis 501a of the wiper lever 419, and a center axis 501d of a wiper axis 500 are positioned on respectively different axes, like in the first embodiment.

Further as illustrated in FIG. 14C, a pivot angle θ3 of the pivot part 509, which corresponds to a pivot angle θ1 of the wiper lever 419, is smaller than a pivot angle θ2 of the wiper axis 500. That is, the pivot angle θ3 between the center axis 501f of the pivot part 509 and the concave 509a as the tip end part of the pivot part 509 pivoted is smaller than the pivot angle θ2 between the center axis 501d of the wiper axis 500 and the tip end part 500a of the wiper axis 500 pivoted in the radial direction.

In the wiper lever 419, when the protrusions 507 are pivoted in the circumferential direction, the pivot part 509 integral with the protrusions 507 are pivoted together. At this time, the wiper axis 500 is also pivoted since the wiper axis 500 is held by the concave 509a. The wiper 417 is thereby pivoted and wipes extraneous matter 418.

Thus, in this embodiment, the wiper lever 419 for operating the wiper 417 can be easily operated even when a harvester 41 as an endoscopic surgical instrument is gripped, as in the first embodiment.

Also in this embodiment, the wiper lever 419 including the protrusions 507 are not all exposed but only the protrusions 507 protrude. Therefore, the wiper 417 is prevented from causing an operation error.

Also in this embodiment, the protrusions 507 and the pivot part 509 are integrated with each other, and the concave 509a is provided at the pivot part 509. Accordingly, pivot force of pivoting the protrusions 507 can be more directly transmitted to the wiper axis 500. In this manner, in this embodiment, the pivot force of pivoting the protrusions 507 can be more easily transmitted to the wiper 417 through the wiper axis 500.

Also in this embodiment, as in the first embodiment, the center axis 501f of the pivot part 509 as the center axis 501a of the wiper lever 419 and the center axis 501d of the wiper axis 500 are positioned on respectively different axes. The pivot angle θ1 of the wiper lever 419 (the pivot angle θ3 of the pivot part 509) is set smaller than the pivot angle θ2 of the wiper axis 500. Therefore, in this embodiment, the wiper 417 can be pivoted over a wide range with a small operation amount of the wiper lever 419 (protrusions 507), as in the first embodiment, and the extraneous matter 418 adhering to the observation surface 54b can be wiped off over the wide range by the wiper 417.

Also in this embodiment, even if a pivot amount (operation amount) of the wiper lever 419 (protrusions 507) is small, the extraneous matter 418 adhering to the observation surface 54b can be wiped off over the wide range, and therefore, labor of operation for pivoting the wiper lever 419 can be saved.

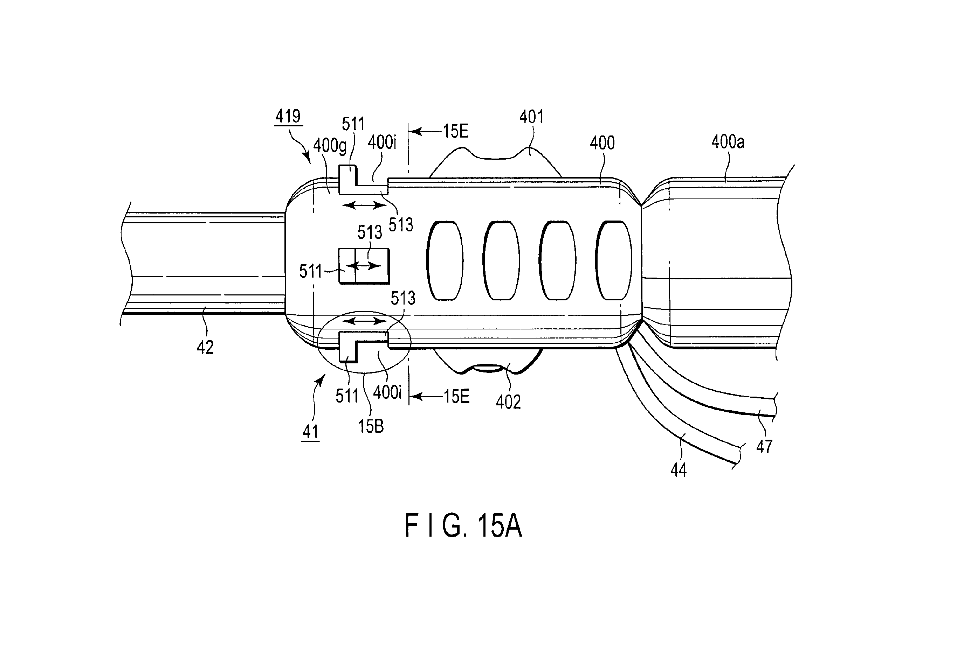

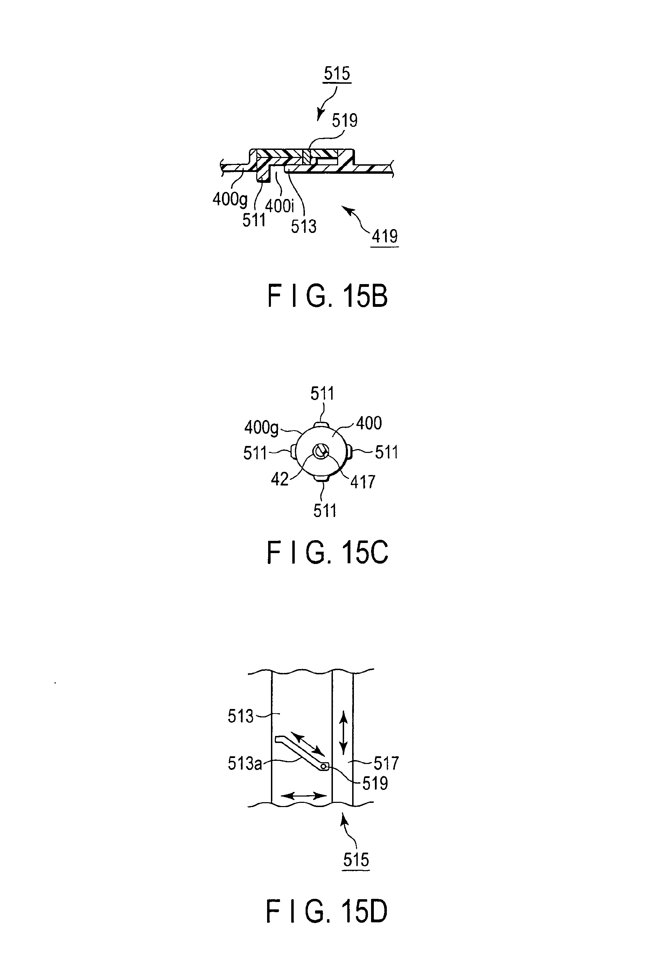

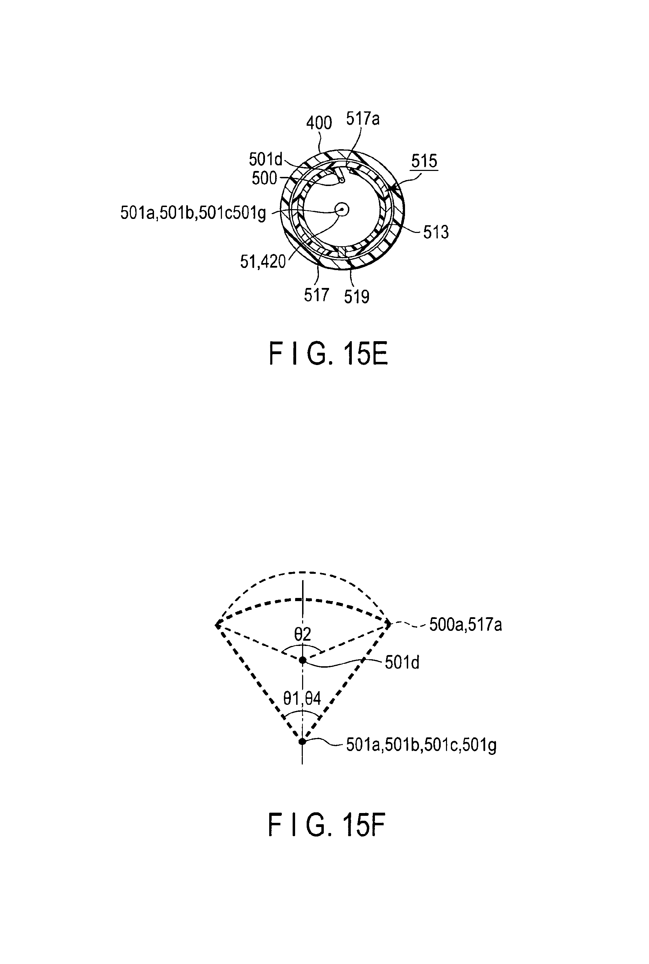

Next, the third embodiment of the invention will be described with reference to FIGS. 15A, 15B, 15C, 15D, 15E, and 15F. The same parts of the configuration as those of the first embodiment described above will be denoted at the same reference symbols, and omitted from descriptions below.

A wiper lever 419 includes plural protrusions 511 which are exposed from an outer surface 400g, provided over the circumferential direction, form knobs for operating the wiper lever 419, and can move forward and backward in the longitudinal direction, a forward/backward movement section 513 which is integral with the protrusions 511 and moves forward and backward inside a grip section 400 in accordance with forward and backward movement of the protrusions 511 and a pivot assembly 515 which is connected to the forward/backward movement section 513, and is pivoted in association with the forward and backward movement of the forward/backward movement section 513, thereby to pivot a wiper axis 500 and to accordingly operate a wiper 417.

The wiper lever 419 according to this embodiment has a ring shape as illustrated in FIG. 15B, and moves forward and backward in the longitudinal direction of the grip section 400. On the wiper lever 419, only the protrusions 511 protrude from the outer surface 400g. The protrusions 511 are provided, for example, at four positions at equal intervals in the circumferential direction, as illustrated in FIG. 15C.

The forward/backward movement section 513 includes a groove 513a formed oblique to the longitudinal direction of the grip section 400, as illustrated in FIG. 15D.

As illustrated in FIGS. 15B and 15E, a pivot assembly 515 includes a pivot press section 517 which operates the wiper 417 in a manner that the pivot press section 517 is pivoted in the circumferential direction in association with forward and backward movement of the forward/backward movement section 513, and accordingly, the wiper axis 500 is pressed in the circumferential direction through a concave 517a sandwiching and holding the wiper axis 500, thereby to pivot the wiper axis 500 and a pin 519 such as a connect section which is engaged in the groove 513a, fixed to the pivot press section 517, connects the pivot press section 517 with the forward/backward movement section 513, and moves in the groove 513a in association with forward and backward movement of the forward/backward movement section 513, thereby to pivot the pivot press section 517 in the circumferential direction.

The pivot press section 517 includes a concave 517a for holding the wiper axis 500. Such a pivot press section 517 has, for example, a substantial C-shape. In other words, the concave 517a is a notch in the pivot press section 517 having the substantial C-shape.

The grip section 400 includes openings 400i from which protrusions 511 protrude. The openings 400i constrain forward and backward movement of the protrusions 511 in the longitudinal direction.

In this embodiment, a center axis 501g of the pivot press section 517, which corresponds to a center axis 501a of the wiper lever 419, and a center axis 501d of the wiper axis 500 are positioned on respectively different axes, as in the first embodiment.

Further, a pivot angle θ4 of the pivot press section 517, which corresponds to a pivot angle θ1 of the wiper lever 419, is smaller than a pivot angle θ2 of the wiper axis 500. That is, the pivot angle θ4 between the center axis 501g of the pivot press section 517 and the concave 517a as the tip end part of the pivot press section 517 pivoted is smaller than the pivot angle θ2 between the center axis 501d of the wiper axis 500 and the tip end part 500a of the wiper axis 500 pivoted in the radial direction.

In the wiper lever 419, when the protrusions 511 move forward and backward, the forward/backward movement section 513 integral with the protrusions 511 accordingly moves forward and backward. At this time, the pin 519 moves in the groove 513a, thereby pivoting the pivot press section 517. At this time, the pivot press section 517 presses the wiper axis 500 in the circumferential direction by the concave 517a, thereby to pivot the wiper axis 500 in the circumferential direction. Accordingly, the wiper 417 is pivoted and wipes extraneous matter 418.

Thus, in this embodiment, the wiper lever 419 for operating the wiper 417 can be easily operated even when the harvester 41 as an endoscopic surgical instrument is gripped.

Also in this embodiment, forward and backward movement can be achieved without pivoting the wiper lever 419. In association with the forward and backward movement, the wiper 417 can be pivoted by the pivot assembly 515.

In other words, according to this embodiment, the wiper 417 can be pivoted by forward and backward movement of the protrusions 511, with the harvester 41 gripped.

Also in this embodiment, only the protrusions 511 protrude, and therefore, the wiper 417 is prevented from causing an operation error.

Also in this embodiment, as in the first embodiment, the center axis 501g of the pivot press section 517 as the center axis 501a of the wiper lever 419, and the center axis 501d of the wiper axis 500 are positioned on respectively different axes. The pivot angle θ1 of the wiper lever 419 (pivot angle θ4 of the pivot press section 517) is set smaller than the pivot angle θ2 of the wiper axis 500. Therefore, in this embodiment, the wiper 417 can be pivoted over a wide range with a small operation amount of the wiper lever 419 (protrusions 511), as in the first embodiment, and the extraneous matter 418 adhering to the observation surface 54b can be wiped off over the wide range by the wiper 417.

Also in this embodiment, even if a forward and backward movement amount (operation amount) of the wiper lever 419 (protrusions 511) is small, the extraneous matter 418 adhering to the observation surface 54b can be wiped off over the wide range, and therefore, labor of operation for pivoting the wiper lever 419 can be saved.

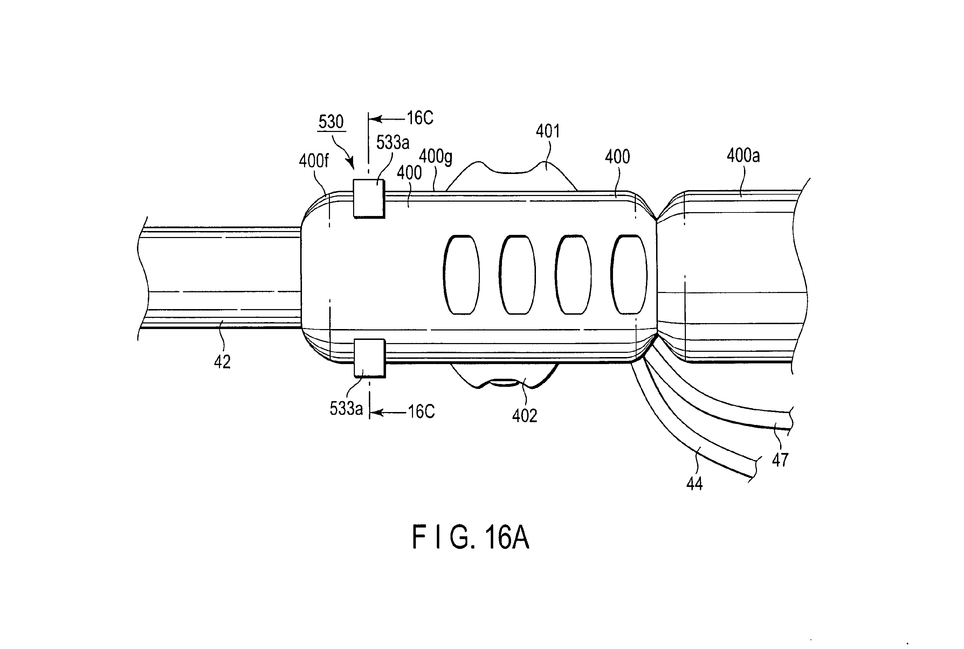

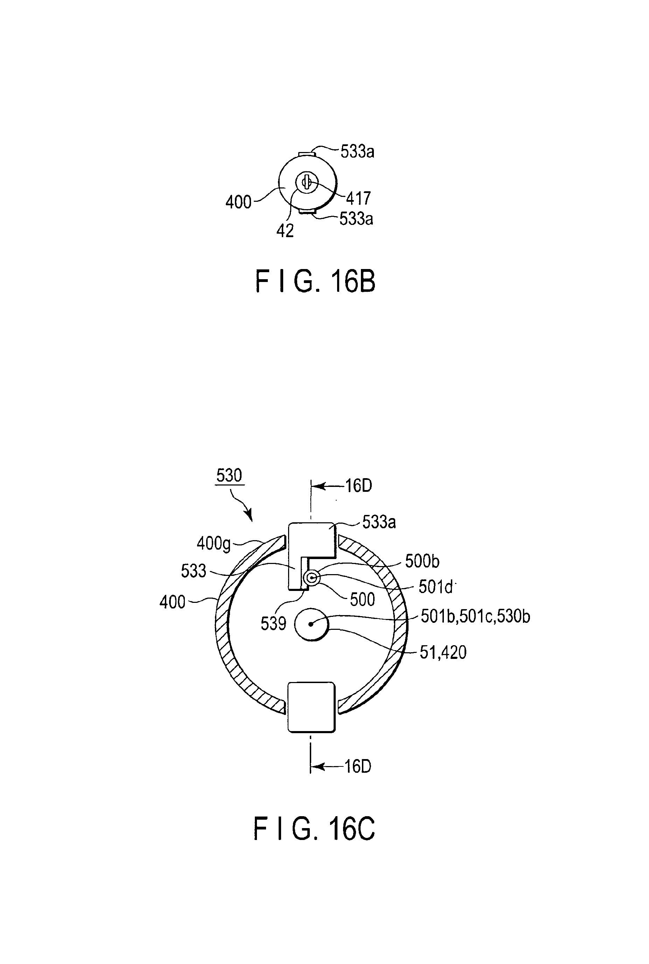

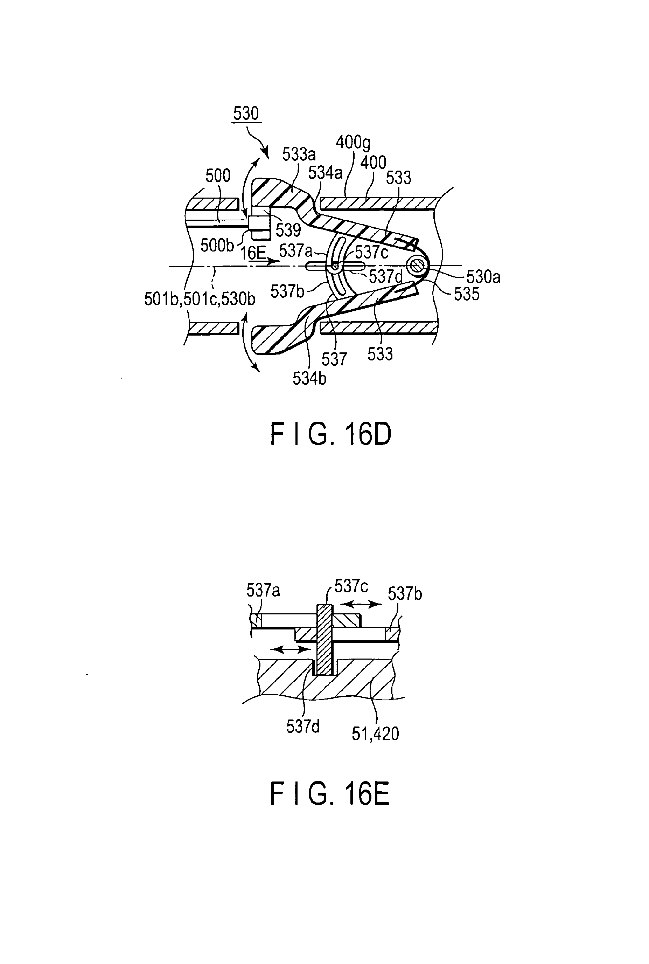

Next, the fourth embodiment according to the invention will be described with reference to FIGS. 16A, 16B, 16C, 16D, and 16E. The same parts of the configuration as those of the first embodiment described above will be denoted at the same reference symbols, and omitted from descriptions given below.

A wiper lever 530 as an operation section in this embodiment is provided at a tip end 400f of a grip section 400, as illustrated in FIG. 16A, and opens/closes in the radial direction of the grip section 400, as illustrated in FIG. 16D. The wiper lever 530 is connected to a wiper axis 500 and opens/close to operate a wiper 417 as a wiping section through the wiper axis 500.

A perpendicular axis 530b, which is perpendicular to an open/close axis 530a of the wiper lever 530 and is provided along the longitudinal direction of an insertion section 42 (harvester 41), is coaxial to a center axis 501b of a rigid endoscope 51 inserted in the harvester 41 and is also coaxial to a center axis 501c of a rigid endoscope insertion channel 420.

The perpendicular axis 530b and a center axis 501d of a wiper axis 500 are positioned on respectively different axes.

As illustrated in FIG. 16D, the wiper lever 530 includes a pair of arm parts 533 constituted by an upper arm 534a and a lower arm 534b wherein a tip end 533a of the upper arm 534a and the lower arm 534b as knobs protrude from an outer surface 400g, an elastic member 535 such as a spring which connects to the arm parts 533 (the upper arm 534a and the lower arm 534b) with each other, includes the open/close axis 530a and opens/closes in the radial direction of the grip section 400 about the open/close axis 530a as a center, a positioning section 537 which positions the wiper lever 530 (the arm parts 533) so that the perpendicular axis 530b is coaxial to a center axis 501b of a rigid endoscope 51 and an operation-section-side engaging section 539 such as a rack, which is provided at the tip end 533a of the arm parts 533 (the upper arm 534a) and positioned inside the grip section 400.

Such a wiper lever 530 is, for example, tongs.

As illustrated in FIGS. 16D and 16E, the positioning section 537 includes a frame 537a provided on the upper arm 534a, a frame 537b provided on the lower arm 534b, a pin 537c as a connection moving part which connects the frames 537a and 537b with each other and moves forward and backward along the longitudinal direction of the insertion section 42 which is a direction perpendicular to the open/close axis 530a, in accordance with opening/closing of the arm parts 533, and a groove 537d which is provided on the outer circumferential surface of the rigid endoscope 51 and the outer circumferential surface of the rigid endoscope insertion channel 420 along the longitudinal direction of the insertion section 42 and allows the pin 537c to move along the longitudinal direction of the insertion section 42 in the groove 537d in accordance with opening/closing of the arm parts 533.

The wiper axis 500 includes an axial-member-side engaging section 500b which is, for example, a pinion engaged with the operation-section-side engaging section 539.

In the wiper lever 530, the arm parts 533 are pressed toward the center (the center axis 501b) of the grip section 400, and open/close about the open/close axis 530a as a center. The operation-section-side engaging section 539 and the axial-member-side engaging section 500b are thereby engaged with each other to pivot the wiper axis 500. The wiper 417 is operated accordingly.

More specifically, as the wiper lever 530 is gripped and pressed, both the upper arm 534a and lower arm 534b are closed (to come close to each other), thereby pivoting the wiper axis 500 together with the axial-member-side engaging section 500b engaged with the operation-section-side engaging section 539. Accordingly, the wiper 417 is pivoted. When the wiper lever 530 is released from hands, the wiper lever 530 opens owing to the elastic member 535, i.e., both the upper arm 534a and lower arm 534b open (to move away from each other). At this time, the wiper axis 500 is pivoted together with the axial-member-side engaging section 500b, in the same manner as described above.

Thus, the wiper axis 500 is pivoted, and accordingly, the wiper 417 is pivoted to wipe off the extraneous matter 418.

When only the lower arm 534b closes (the lower arm 534b comes closes to the upper arm 534a), the frame 537b moves toward the upper arm 534a. In this manner, the pin 537c energized by the frames 537a and 537b is released, as illustrated in FIG. 16D. At this time, the pin 537c is pressed toward the open/close axis 530a in the groove 537d. The pin 537c further moves along the longitudinal direction of the insertion section 42 in the groove 537d, thereby adjusting the position of the pin 537c in the groove 537a. The pin 537c is then pressed the frame 537a toward the lower arm 534b, thereby closing the upper arm 534a. In this manner, the wiper axis 500 is pivoted together with the axial-member-side engaging section 500b engaged with the operation-section-side engaging section 539, and accordingly, the wiper 417 is thereby pivoted.

When only the upper arm 534a is closed, the frame 537a presses the pin 537c toward the open/close axis 530a in the groove 537a. The pin 537c then moves along the longitudinal direction of the insertion section 42 in the groove 537d, thereby adjusting the position of the pin 537c in the groove 537d. Accordingly, a closed position of the wiper lever 530 (the arm parts 533) is determined, and the wiper 417 is pivoted.

From the state as described above, the wiper lever 530 (the arm parts 533) opens due to the elastic force of the elastic member 535 (the lower arm 534b moves away from the upper arm 534a, and the upper arm 534a moves away from the lower arm 534b), and accordingly, the frame 537b (the frame 537a) moves away from the upper arm 534a (the lower arm 534b). The released pin 537c is thereby actuated by the frames 537a and 537b, as illustrated in FIG. 16D. At this time, the lower arm 534b (the frame 537b) presses the pin 537c in a direction (toward the side of the wiper axis 500) opposite to the open/close axis 530a in the groove 537d, due to recovery force of the lower arm 534b. The pin 537c accordingly moves in the longitudinal direction of the insertion section 42 in the groove 537d, thereby adjusting the position of the pin 537c in the groove 537d. In this manner, an opened position of the wiper lever 530 is determined in the same manner as described above, and the wiper axis 500 is pivoted together with the axial-member-side engaging section 500b engaged with the operation-section-side engaging section 539. Accordingly, the wiper 417 is pivoted.

Thus, in this embodiment, the wiper lever 419 for operating the wiper 417 can be easily operated even when the harvester 41 as an endoscopic surgical instrument is gripped.

Also in this embodiment, the wiper lever 419 can be easily operated by simply gripping or releasing the wiper lever 530.

Also in this embodiment, the wiper 417 can be pivoted by operating either both of the upper arm 534a and lower arm 534b or only the lower arm 534b.

That is, in this embodiment, the wiper 417 can be pivoted by open/close operation of the arm parts 533, with the harvester 41 gripped.

Thus, the present invention is not directly limited to the embodiments described above but various modifications to components of the invention are available in practical phases without deviating from the subject matter of the invention. Various inventions can further be derived from appropriate combinations of plural components disclosed in the above embodiments.

## Claims

1. An endoscopic surgical instrument (41) comprising:
an insertion section (42) to be inserted into a body cavity;
a grip section (400) provided to be linked with a base end of the insertion section (42);
a wiping section (417) that is provided at a tip end of the insertion section (42) and is pivoted to wipe off extraneous matter (418) adhering to an observation surface (54b) provided at a tip end part of a lens (54b);
an axial member (500) that is inserted through the insertion section (42) and is connected to the wiping section (417); and
an operation section (419) that is provided over a whole circumference of a tip end (400f) of the grip section (400) in a circumferential direction in a longitudinal direction of the grip section (400), is connected to the axial member (500), and operates the wiping section (417) through the axial member (500), wherein
a center axis (501a) of the operation section (419) is coaxial to a center axis (501b) of an endoscope (51) when the endoscope (51) inserted through the grip section (400) and the insertion section (42).

2. The endoscopic surgical instrument (41) according to claim 1, wherein the center axis (501a) of the operation section (419) and a center axis (501d) of the axial member (500) are positioned on respectively different axes.

3. The endoscopic surgical instrument (41) according to claim 2, wherein the operation section (419) is provided at a position where the operation section (419) is able to be operated with the grip section (400) gripped.

4. The endoscopic surgical instrument (41) according to claim 3, wherein the operation section (419) is pivoted in the circumferential direction, thereby to pivot the axial member (500) which is connected to the operation section (419) in the circumferential direction, and to accordingly operate the wiping section (417).

5. The endoscopic surgical instrument (41) according to claim 4, wherein a pivot angle of the operation section (419) is smaller than a pivot angle of the axial member (500).

6. The endoscopic surgical instrument (41) according to claim 5, wherein the operation section (419) includes a holder section (503) that holds the axial member (500) to connect to the axial member (500), the holder section (503) being provided on an inner circumferential surface (419b) of the operation section (419).

7. The endoscopic surgical instrument (41) according to claim 6, wherein the holder section (503) has a cam that holds the axial member (500) by pinching an end of the axial member (500).

8. The endoscopic surgical instrument (41) according to claim 7, wherein the operation section (419) has a tapered shape.

9. The endoscopic surgical instrument (41) according to claim 8, wherein
the operation section (419) includes a plurality of protrusions (505) to form knobs for operating the operation section (419), the plurality of protrusions (505) being provided on an outer circumferential surface (419c) of the operation section (419) over the circumferential direction, and
the operation section (419) including the protrusions (505) is exposed outside.

10. The endoscopic surgical instrument (41) according to claim 9, wherein tip ends (505a) of the protrusions (505) are located inside of an outer surface (400g) of the grip section (400), in radial directions of the grip section (400).

11. The endoscopic surgical instrument (41) according to claim 3, wherein
the operation section (419) includes:
a plurality of protrusions (507) that are exposed from an outer surface (400g) of the grip section (400), provided over the circumferential direction, form knobs for operating the operation section (419), and are pivotable in the circumferential direction; and
a pivot part (509) that has a concave (509a) for holding the axial member (500), is integrated with the plurality of protrusions (507), is provided in the grip section (400), and is pivoted in association with pivot operation of the plurality of protrusions (507), thereby to pivot the axial member (500) through the concave (509a) and to accordingly operate the wiping section (417), and
the grip section (400) has openings (400h) from which the plurality of protrusions (507) protrude thereby to constrain pivoting of the plurality of protrusions (507) in the circumferential direction.

12. The endoscopic surgical instrument (41) according to claim 11, wherein a pivot angle of the pivot part (509) is smaller than a pivot angle of the axial member (500).

13. The endoscopic surgical instrument (41) according to claim 3, wherein
the operation section (419) includes:
a plurality of protrusions (511) that are exposed from an outer surface (400g) of the grip section (400), provided over the circumferential direction, form knobs for operating the operation section (419), and are movable forward and backward in the longitudinal direction;
a forward/backward movement section (513) that is integral with the plurality of protrusions (511), and moves forward and backward in the grip section (400) in accordance with forward and backward movement of the plurality of protrusions (511); and
a pivot assembly (515) that is connected to the forward/backward movement section (513), and is pivoted in association with the forward and backward movement of the forward/backward movement section (513), thereby to pivot the axial member (500) and to accordingly operate the wiping section (417), and
the grip section (400) has openings (400i) from which the plurality of protrusions (511) protrude thereby to constrain forward and backward movement of the plurality of protrusions (511) in the longitudinal direction.

14. The endoscopic surgical instrument (41) according to claim 13, wherein
the forward/backward movement section (513) includes a groove (513a) formed oblique to the longitudinal direction,
the pivot assembly (515) includes:
a pivot press section (517) that has a concave (517a) for holding the axial member (500), and is pivoted in the circumferential direction in association with the forward and backward movement of the forward/backward movement section (513), thereby to press the axial member (500) in the circumferential direction through the concave (517a), pivoting the axial member (500), and to accordingly operate the wiping section (417); and
a connect section (519) that is engaged in the groove (513a), is fixed to the pivot press section (517), connects the pivot press section (517) with the forward/backward movement section (513), and moves in the groove (513a) in association with the forward and backward movement of the forward/backward movement section (513), thereby to pivot the pivot press section (517) in the circumferential direction.

15. The endoscopic surgical instrument (41) according to claim 14, wherein a pivot angle of the pivot press section (517) is smaller than a pivot angle of the axial member (500).

16. An endoscopic surgical instrument (41) comprising:
an insertion section (42) to be inserted into a body cavity;
a grip section (400) provided to be linked with a base end of the insertion section (42);
a wiping section (417) that is provided at a tip end of the insertion section (42) and is pivoted to wipe off extraneous matter (418) adhering to an observation surface (54b) provided at a tip end (54a) of an endoscope (51);
an axial member (500) that is inserted through the insertion section (42) and is connected to the wiping section (417); and
an operation section (530) that is provided at a tip end (400f) of the grip section (400), opens and closes in a radial direction of the grip section (400), is connected to the axial member (500), and operates the wiping section (417) through the axial member (500) by opening and closing, wherein
a perpendicular axis (530b) which is perpendicular an open/close axis (530a) of the operation section (530) and is provided along a longitudinal direction of the insertion section (42) is coaxial to a center axis (501b) of the endoscope (51).

17. The endoscopic surgical instrument (41) according to claim 16, wherein
the operation section (530) includes:
a pair of arm parts (533) having tip ends (533a) to form knobs protruding from the grip section (400);
an elastic member (535) that connects to the arm parts (533) with each other, has the open/close axis (530a), and opens/closes in the radial direction of the arm parts (533) about the open/close axis (530a) as a center;
a positioning section (537) that positions the arm parts (533) in a manner that the perpendicular axis (530b) is coaxial to the center axis (501b) of the endoscope (51); and
an operation-section-side engaging section (539) that is provided at a tip end (533a) of the arm parts (533), so as to be located inside the grip section (400);
the axial member (500) has an axial member-side-engaging section (500b) engaged with the operation section-side-engaging section(539), and
the arm parts (533) open and close about the open/close axis (530a) as a center, thereby to engage the operation section-side-engaging section (539) with the axial member-side-engaging section (500b), to pivot the axial member (500), and to accordingly operate the wiping section (417).

18. An endoscopic surgical instrument (41) comprising:
an insertion section (42) to be inserted into a body cavity;
a grip section (400) provided to be linked with a base end of the insertion section (42);
a wiping section (417) that is provided at a tip end of the insertion section (42) and is pivoted to wipe off extraneous matter (418) adhering to an observation surface (54b);
an axial member (500) that is inserted through the insertion section (42) and is connected to the wiping section (417); and
an operation section (419) that is provided over a whole circumference of a tip end (400f) of the grip section (400) in a circumferential direction in a longitudinal direction of the grip section (400), is connected to the axial member (500), and operates the wiping section (417) through the axial member (500), wherein
a center axis (501a) of the operation section (419) and a center axis (501d) of the axial member (500) are positioned on respectively different axes.

19. The endoscopic surgical instrument (41) according to claim 18, wherein the operation section (419) is provided at a position where the operation section (419) is able to be operated with the grip section (400) gripped.

20. The endoscopic surgical instrument (41) according to claim 19, wherein the operation section (419) is pivoted in the circumferential direction, thereby to pivot the axial member (500) which is connected to the operation section (419) in the circumferential direction, and to accordingly operate the wiping section (417).

21. The endoscopic surgical instrument (41) according to claim 20, wherein a pivot angle of the operation section (419) is smaller than a pivot angle of the axial member (500).
